# EUROPEAN PATENT SPECIFICATION

(11) **EP 3 013 471 B1**
(45) Date of publication and mention of the grant of the patent: **27.12.2017**
(21) Application number: 14735830.3
(22) Date of filing: 17.06.2014
(51) Int. Cl.: B01J 21/08, C07C 67/327

(54) **PROCESS FOR PRODUCTION OF METHACRYLIC ACID ESTERS**
VERFAHREN ZUR HERSTELLUNG EINES METHACRYLSÄUREESTERS
PROCÉDÉ DE PRODUCTION D'ESTERS D'ACIDE MÉTHACRYLIQUE

(30) Priority: 26.06.2013 US 201361839590 P
(43) Date of publication of application: 04.05.2016
(73) Proprietor: Rohm and Haas Company, Philadelphia, PA 19106 (US)
(72) Inventor: LEMONDS, Andrew M., Lake Jackson, TX 77566 (US); XU, Jinsuo, Fort Washington, PA 19034 (US)
(74) Representative: Boult Wade Tennant
(86) International application number: PCT/US2014/042666
(87) International publication number: WO 2014/209672

(56) References cited:
- WO-A1-2012/047883

## Description

### BACKGROUND OF THE INVENTION

The invention relates to a catalytic process for producing α-, β-unsaturated carboxylic acid esters from acetone cyanohydrin and sulfuric acid.

A number of commercial processes are practiced for the production of such esters, including sulfuric acid treatment of acetone cyanohydrin ("ACH"), two stage oxidation of isobutylene or t-butyl alcohol, and liquid phase catalytic condensation of propionaldehyde with formaldehyde.

U.S. 4,529,816 describes a conventional process for the production of methyl methacrylate ("MMA") from ACH. In this process, ACH is hydrolyzed by sulfuric acid to produce α-hydroxyisobutyramide ("HIBAM") and α-sulfatoisobutyramide ("SIBAM"). Next, the HIBAM and SIBAM are thermally converted to 2-methacrylamide ("MAM") and a small amount of methacrylic acid ("MAA"). The MAM is esterified with methanol to produce the desired MMA product, while residual HIBAM is esterified to methyl α-hydroxyisobutyrate ("α-MOB"). The esterification product stream is a mixed product that is subjected to separation and purification steps to isolate the MMA product from the other compounds. Typically, a purified MMA product stream is produced, along with a purification residue comprising other compounds including, but not limited to, α-MOB and methyl β-methoxyisobutyrate (β-MEMOB). The recovery and conversion of one or more of these other compounds to produce additional MMA product has been the subject of various research and development efforts having varying degrees of success and practical utility. In particular, U.S. 4,529,816 describes an improvement wherein the α-MOB is isolated and recycled to the process between the thermal conversion and esterification steps.

A variety of solid catalysts have been used for converting α-MOB and/or β-MEMOB into MMA and MAA in the vapor phase. For example, in Japanese Patent Publication Nos. 20611/1969, 20612/1969 and 15724/1970, a phosphate-based acid or salt deposited onto silica or silica-alumina was used. These technologies were plagued by the need for very high reaction temperatures, unacceptable levels of by-product methyl isobutyrate (MIB) formation, and fast deactivation by coke deposition. Crystalline aluminosilicates containing alkali or alkaline earth metals have been thoroughly studied for the conversion of α-MOB and β-MEMOB into MMA and MAA, as disclosed in U.S. 5,371,273, U.S. 5,393,918, and U.S. 5,739,379, as well as JP Application No. 65896/1990, U.S. 5,250,729, 5,087,736 and EP 429,800 A2. The dehydration of α-MOB to MMA was commercialized by the Mitsubishi Gas Chemical Company in 1997 as a sulfuric acid-free ACH-based MMA process. The art shows that crystalline aluminosilicates such as zeolite NaX are well suited for α-MOB dehydration; however, they are limited in their ability to achieve simultaneous high yields on α-MOB and β-MEMOB and, therefore, have limited applicability for MMA residue yield recovery.

Catalysts containing Cs and silica gels have been explored for a number of reactions, including dehydrations, aldol condensations and Michael additions, other than conversion of α-MOB and/or β-MEMOB into MMA and MAA in the vapor phase. U.S. 4,841,060, U.S. 5,625,076, and U.S. 5,304,656, for example, disclose catalysts containing silicon and at least one element selected from the group consisting of alkali metals and alkaline earth metals for intramolecular dehydrations, such as the conversion of mercaptoalkanols to alkylene sulfides, alkanolamines to cyclic amines, N-(2-hydroxyethyl)-2-pyrrolidone to N-vinyl-2-pyrrolidone, and tertiary N-(2-hydroxyalkyl) carboxylic acid amide to tertiary N-alkenyl carboxylic acid amide. The substrates and reactions involved in these processes, however, differ chemically from dehydration and demethanolation of α-MOB and β-MEMOB, respectively, to MMA.

WO 2012/047883 discloses a method for producing the desired esters via the catalytic conversion of ACH process by-products using a catalyst comprising at least one Group 1A element.

It would be desirable to have an improved catalytic process for producing the desired esters.

### SUMMARY OF THE INVENTION

The process of the invention is such a process, the process comprising the steps of:
providing a C₁-C₁₂ alkyl alcohol and an organic fraction comprising C₁-C₁₂ alkyl methacrylate, C₁-C₁₂ alkyl α-hydroxyisobutyrate and C₁-C₁₂ alkyl β-C₁-C₁₂ alkoxyisobutyrate;
vaporizing at least a portion of the organic fraction and at least a portion of the C₁-C₁₂ alkyl alcohol;
contacting the vaporized organic fraction and alcohol with a catalyst to convert the C₁-C₁₂ alkyl α-hydroxyisobutyrate and C₁-C₁₂ alkyl β-C₁-C₁₂ alkoxyisobutyrate to additional C₁-C₁₂ alkyl methacrylate and produce a converted mixture that comprises a C₁-C₁₂ alkyl methacrylate, methacrylic acid, C₁-C₁₂ alkyl alcohol, and water, wherein the catalyst (1) comprises at least one element selected from the group consisting of lithium, sodium, potassium, rubidium, cesium and francium, (2) is supported on a support comprising porous silica, and (3) comprises from 0.005 to 5 moles of boron as a promoter per 100 moles of silica.

Surprisingly, the use of the catalyst having boron as a promoter or dopant, such as boron in the form of boric acid, unexpectedly improves the catalyst stability against deactivation.

### BRIEF DESCRIPTION OF THE DRAWINGS

Figure 1 is a graph comparing the performance of Zr- and Bi-promoted catalysts vs. a non-promoted catalyst
Figure 2 is a graph comparing the performance of boron-promoted catalysts vs. a non-promoted catalyst.

### DETAILED DESCRIPTION OF THE INVENTION

Also herein, the recitations of numerical ranges by endpoints include all numbers subsumed in that range (e.g., 1 to 5 includes 1, 1.5, 2, 2.75, 3, 3.80, 4, 5, etc.). For the purposes of the invention, it is to be understood, consistent with what one of ordinary skill in the art would understand, that a numerical range is intended to include and support all possible subranges that are included in that range. For example, the range from 1 to 100 is intended to convey from 1.01 to 100, from 1 to 99.99, from 1.01 to 99.99, from 40 to 60, from 1 to 55, etc.

As used herein, the use of the term "(meth)" followed by another term such as acrylate refers to both acrylates and methacrylates. For example, the term "(meth)acrylate" refers to either acrylate or methacrylate; the term "(meth)acrylic" refers to either acrylic or methacrylic; and the term "(meth)acrylic acid" refers to either acrylic acid or methacrylic acid.

The invention relates to an improved process targeted for converting some side products, recovered from the purification residue of methyl methacrylate (MMA) product, to MMA in a heterogeneous phase reaction. The side products generated from a conventional acetone cyanohydrin (ACH) process, may include methyl α-hydroxyisobutyrate (a-MOB), methyl β-methoxyisobutyrate (β-MEMOB), methacrylic acid (MAA), methyl β-hydroxyisobutyrate (β-MOB), in addition to methacrylamide (MAM), MMA dimer, and other unknown heavies. The reactions giving MMA from heavies include the following:

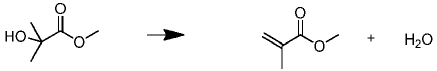

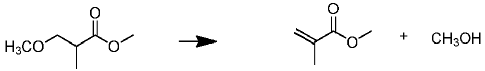

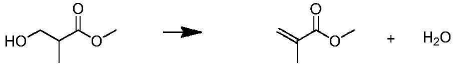

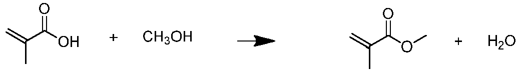

The process of the invention employs a supported catalyst comprising boron as a promoter and at least one alkali metal element selected from the group consisting of lithium, sodium, potassium, rubidium, cesium and francium. The element may be in any form suitable for use as a catalyst under the conditions in the reactor, e.g., it may be present as a compound of the element and another element. In one embodiment of the invention, the element of the catalyst may be present as a metal oxide, hydroxide or carbonate. In one embodiment of the invention, the boron promoter of the catalyst may be present as a boron oxide or hydroxide. In one embodiment of the invention, the amount of boron can be in the range of 0.01 to 1.0 wt% of the whole catalyst mass. The amount of alkali metal, preferably cesium, can be in the range of 1.0 to 30.0 wt%, preferably in the range of 2.0 -15.0 wt% based on the weight of the whole catalyst mass. In various embodiments of the invention, the catalyst comprises from 0.005 to 5 moles boron as a promoter per 100 moles of the silica in the support, or from 0.010 to 4 moles per 100 moles of the silica, or from 0.1 to 1 moles per 100 moles of the silica. In one embodiment of the invention, the amount of boron is from 0.005 to less than 0.25 moles per 100 moles of the silica.

The catalyst support advantageously is a porous support. The catalyst preferably comprises a porous siliceous support material having pore openings greater than 1 nanometer. The "pore opening" or "pore size" or "average pore size" as used herein mean the average diameter of the pore opening, which is determined using the well-known BET nitrogen adsorption or desorption method. See S. Brunauer et al., J.A.C.S., 60, 309, (1938)]. The porous support comprises silica and can be essentially all silica or can include other materials, such as alumina, titania, magnesia, calcium oxide, active carbon, and combinations thereof. The silica may be silica gel, fumed silica, or colloidal silica, in their pure forms, or in a combination of two or more. A silica gel type of material is preferred due to its weak acid-base property and high surface area. Some experimental silica materials, such as mesoporous silica and foam silica like MCM-41, SBA-15, as disclosed in the literature (Nature, 1985, 318, 162; Science, 1998, 279, 548), can also be used. The chosen support material should provide good distribution for the alkali metal and the promoter, and should not interfere with the desired reaction(s).

The recovery process of the invention converts certain by-product species to MMA. For example, a stream enriched in by-products is obtained by distillation of a residue stream and is subjected to the vapor phase catalytic reaction process described herein. In one embodiment, the invention is a process for producing high purity α, β-unsaturated carboxylic acid esters in high yield, based on the starting ACH. The purity of the ester product preferably is greater than about 99 weight percent, although less pure products can be obtained from the process if desired. The yield of product esters from the process preferably is greater than about 95 percent, based on the starting ACH. In one embodiment of the invention, the yield is at least 2, preferably at least 4, percent higher than that of a prior art process having no post-reactor containing the catalyst employed in the inventive process.

In one embodiment of the invention, the inventive process involves the following steps:
(a) Hydrolyze ACH with sulfuric acid to produce a hydrolysis mixture comprising 2-methacrylamide, α-sulfatoisobutyramide, α-hydroxyisobutyramide, and methacrylic acid;
(b) Esterify the hydrolysis mixture with a C₁-C₁₂ alkyl alcohol to produce an esterification mixture comprising a C₁-C₁₂ alkyl methacrylate, a C₁-C₁₂ alkyl α-hydroxyisobutyrate and C₁-C₁₂ alkyl β- C₁-C₁₂ alkoxyisobutyrate;
(c) Separate the esterification mixture into an aqueous fraction and an organic fraction comprising C₁-C₁₂ alkyl methacrylate, C₁-C₁₂ alkyl α-hydroxyisobutyrate and C₁-C₁₂ alkyl β-C₁-C₁₂ alkoxyisobutyrate;
(d) Provide a C₁-C₁₂ alkyl alcohol co-feed (which may or may not be the same alcohol as the C₁-C₁₂ alkyl alcohol used in the esterifying step (b));
(e) Vaporize the co-feed and at least a portion of the organic fraction to produce a vapor feed stream; and
(f) Contact the vapor feed stream with a catalyst of the invention, to convert the C₁-C₁₂ alkyl α-hydroxyisobutyrate and C₁-C₁₂ alkyl β-C₁-C₁₂ alkoxyisobutyrate to additional C₁-C₁₂ alkyl methacrylate to a converted mixture comprising the C₁-C₁₂ alkyl methacrylate, methacrylic acid, C₁-C₁₂ alkyl alcohol, and water. The converted mixture may be wholly or partially recycled.

In one embodiment of the invention, the HIBAM concentration in the process stream just prior to esterification is from about 2 to about 20 mole% based on the starting ACH. In one embodiment of the invention, the SIBAM concentration in the process stream just prior to esterification is from about 1 to about 20 mole% based on the starting ACH.

It is noted that the conversion that occurs during the contacting step, e.g., step (f), involves concurrent dehydration of C₁-C₁₂ alkyl α-hydroxyisobutyrate to additional C₁-C₁₂ alkyl methacrylate and demethanolation of C₁-C₁₂ alkyl β-C₁-C₁₂ alkoxyisobutyrate to additional C₁-C₁₂ alkyl methacrylate. Thus, by-products are recovered and simultaneously converted to additional desired C₁-C₁₂ alkyl methacrylate product. The process of the invention involves conversion of the by-products prior to recycling to the process, and in the process a greater portion of the recovered by-products can be converted, compared to previously practiced processes.

The recovery process of the invention converts distillation residue species to MMA. The organic fraction of the separation step, e.g., step (c), at a minimum, comprises C₁-C₁₂ alkyl methacrylate, C₁-C₁₂ alkyl α-hydroxyisobutyrate and C₁-C₁₂ alkyl β-C₁-C₁₂ alkoxyisobutyrate. For example, the C₁-C₁₂ alkyl methacrylate may be MMA, the C₁-C₁₂ alkyl α-hydroxyisobutyrate may be α-MOB, the C₁-C₁₂ alkyl β-C₁-C₁₂ alkoxyisobutyrate may be β-MEMOB, and in this case the organic fraction comprises the MMA, α-MOB and β-MEMOB. The organic fraction may also comprise organic acids such as, for example, MAA.

Depending on the configuration of the process equipment, the organic fraction may contain varying amounts of C₁-C₁₂ alkyl methacrylate. For example, in one embodiment the portion of the organic fraction fed to the vaporizer may comprise, for example, from 20 to 70 weight percent of C₁-C₁₂ alkyl methacrylate. In other embodiments the portion of the organic fraction fed to the vaporizer may comprise, for example, from 0 to 5, or 0 to 10, weight percent of C₁-C₁₂ alkyl methacrylate.

The co-feed C₁-C₁₂ alkyl alcohol preferably is methanol. Advantageously, the alcohol is employed in an amount sufficient to maintain a relatively high ratio of MMA to MAA in the reactor product stream. Preferably, the weight ratio of co-feed to organic fraction fed to the reactor is from 0.2 to 2.

For illustrative purposes, the following description will focus on a method for producing methyl methacrylate (MMA) as the C₁-C₁₂ alkyl methacrylate. However, as will be readily recognized by persons of ordinary skill in the relevant art, the method of the present invention is applicable to preparation of methacrylic acid esters via the sulfuric acid/ACH process and esterification with C₁-C₁₂ alkyl alcohols. Generally, use of alcohols of C₁-C₄, such as any of methanol, ethanol, n-propanol, isopropanol, n-butanol, and isobutanol, is most common because of the commercial value of the resulting methacrylate esters. Methanol is the preferred alcohol.

The process of the invention can be employed with various MMA production processes, e.g., those disclosed in WO 2012/047883. For example, in one embodiment of the invention a crude MMA stream is obtained by the conventional ACH route to MMA and comprises a mixture of MMA and heavy ends. The crude MMA stream is distilled in an MMA product column under conventional conditions known to those skilled in the art, to yield a high purity, product grade MMA distillate and an MMA product column bottoms stream comprising the heavy ends and some residual MMA. The MMA product column bottoms stream may contain, for example, about 50 wt.% MMA and about 50 wt.% heavy ends. According to this embodiment, the MMA product column bottoms stream is the portion of the organic fraction that is vaporized in a vaporizer together with a methanol vaporizer feed stream. The vaporizer effluent stream comprising vaporized MMA product column bottoms stream and vaporized methanol is fed to a recovery reactor, wherein α-MOB, β-MEMOB, MAA, and methyl β-hydroxyisobutyrate β-MOB) present in the product column bottoms stream are converted to MMA.

In another embodiment of the invention, the MMA product column bottoms stream is further distilled in an MMA recovery column to remove overhead a substantial amount of the residual MMA, yielding a heavy residue stream. The heavy residue stream is substantially depleted in MMA. For example, it may contain 5 wt.% MMA or less. The heavy residue stream is vaporized in the vaporizer. Thus, the heavy residue stream is the portion of the organic fraction that is vaporized in the vaporizer together with the methanol vaporizer feed stream. According to this embodiment, the vaporizer effluent stream comprising the vaporized heavy residue stream and vaporized methanol are fed to the recovery reactor, wherein α-MOB, β-MEMOB, MAA, and β-MOB are converted to a converted mixture comprising MMA, which mixture is discharged from the reactor in a values stream.

In another embodiment of the invention the heavy residue stream is fed to a flash distillation apparatus. For example, 50 to 80 wt.% of the heavy residue stream is evaporated in the flash distillation apparatus, while the remainder exits the flash distillation apparatus in a liquid phase stripped heavy residue stream, which can be processed further or treated as waste or fuel. When the flash distillation is conducted under vacuum, e.g., at 3.33 to 6.67 kPa (25 to 50 mmHg absolute), the flash temperature preferably is in the range of 120 to 150°C. The flash evaporated heavies are then condensed in a condenser and the condensed heavies stream is subsequently vaporized in the vaporizer, then fed, as a co-feed with vaporized methanol, to the recovery reactor, wherein α-MOB, β-MEMOB, MAA, and β-MOB are converted to MMA, which is discharged from the reactor in the values stream. Thus, the condensed heavies stream is the portion of the organic fraction that is vaporized in the vaporizer for this embodiment. For this embodiment, the flash distillation apparatus advantageously is operated at or below, preferably at, a temperature beyond which incremental MMA recovery is not achieved or is negligible.

An alternative embodiment to that of the previous paragraph involves sending the flash evaporated heavies to the recovery reactor, without passing the heavies through the condenser. The heavies may be sent to the reactor either with or without sending them through the vaporizer. It is possible in this embodiment to replace to replace condenser with a compressor to transfer the flash evaporated heavies vapor stream to the vaporizer. Operating conditions for these embodiments can be readily determined by those skilled in the art.

In one embodiment of the present invention, ACH is hydrolyzed using excess sulfuric acid at a temperature from about 80°C to about 135°C, preferably from about 80°C to about 105°C, for a time sufficient to maximize the pre-esterification yield of the total of MAM, SIBAM, HIBAM, and MAA. The temperature can be maintained at a single value or changed during the course of the reaction. This may be accomplished either continuously or stepwise. The time required will vary from less than 1 minute to about 60 minutes and a hydrolysis mixture will be produced comprising MAM, SIBAM, HIBAM, and MAA. Sulfuric acid solution concentrations of 95-100% or more are preferred, but 100% or higher, e.g., oleum, sulfuric acid is not required. The mole percent distribution of reacted ACH equivalent products in the resulting hydrolysis mixture will vary. However, conditions are preferred that result in the following composition: about 60-80% MAM; about 1-20% SIBAM; about 2-20% HIBAM (more preferably 5-15%); and about 0-5% MAA with an overall ACH conversion rate of about 100%. One advantage of this embodiment is that the yield loss is reduced compared to losses incurred in the conventional process by efforts to reduce HIBAM levels during thermal conversion to MAM.

In one embodiment of the invention, the flash distillation apparatus is replaced by a multistage fractional distillation apparatus. For example, 40 to 60 wt.% of the heavy residue stream is distilled in the fractional distillation apparatus, while the remainder that contains MAA, MAM and others exits the fractional distillation apparatus in a liquid phase stripped heavy residue stream, which can be processed further or treated as waste or fuel. The distillation is preferably, but not limited to, a multi-stage, vacuum distillation, which may be conducted batchwise or continuously. For example, a suitable continuous distillation method comprises vacuum distilling using a 10 to 30 tray tower, where the reboiler pressure is in the range of 25 to 200 Torr (3.33 to 26.66 kPa). Preferably, the reboiler pressure is about 150 Torr (20.0 kPa) or less and, depending on the bottoms composition, a reboiler temperature of 150°C or less is obtained. The distillate-to-feed (D/F) and reflux (L/D) ratios are selected based on the feed composition and desired species recoveries according to methods known to those skilled in the art. Representative D/F and L/D ratios are, respectively, from 0.2 to 0.6 and from 0.4 to 1.0. For this embodiment, the fractional distillation apparatus preferably is operated at a temperature such that heavies, such as MAA and MAM, do not get into the distillate stream.

The use of a polymerization inhibitor in the column is desirable to prevent thermally-induced polymerization of present olefinic species. Many polymerization inhibitors are known to those skilled in the art. Combinations of inhibitors can be employed. An example of an effective inhibitor is phenothiazine (PTZ), which can be introduced at the top of the column. The inhibitor may be delivered in any suitable fashion such as, for example, as a solution in MMA, in a composition similar to the distillate, or in a fraction of the distillate itself. An effective inhibitor level provides about 150 ppm PTZ in the column bottoms stream. When using other inhibitors, different concentrations may be required, as is known to those skilled in the art. The distillation overhead stream is then fed to a condenser and the process continues as described hereinabove.

Optionally, the method of the invention may include the aforementioned thermal conversion step after the hydrolyzing step and prior to the esterifying step, wherein at least a portion of the HIBAM in the hydrolysis mixture is converted to MAM, and the resulting cracked hydrolysis mixture is provided to the esterifying step. When practiced, the thermal conversion step comprises heating the hydrolysis mixture to between 90°C and 160°C to convert the HIBAM and SIBAM to MAM and produce the cracked hydrolysis mixture that comprises less HIBAM and more MAM than the original hydrolysis mixture.

The invention makes the thermal conversion step of the old conventional ACH process an optional step. The typically harsh conditions needed to maximize the MAM yield in the thermal conversion step also served to reduce the overall yield of the process due to side reactions such as, for example, the decomposition of MAM and any MAA, or the dimerization of MAM, and the like. By reducing the severity of the thermal conversion conditions, the yield of MAM may also be reduced due to the lower conversion of SIBAM and HIBAM to MAM. However, in subsequent steps of the method of the present invention, any excess SIBAM and HIBAM is esterified into α-MOB, which is then concurrently converted in the presence of the above-described catalyst to additional MMA. Regardless of whether thermal conversion is employed, additional MMA is produced and recycled to the process, providing an overall increase in the yield of MMA from the process as well as a reduction of waste material that must be disposed of by incineration, landfilling, or the like.

The hydrolysis mixture (uncracked or cracked), comprising MAM, SIBAM, HIBAM and MAA, is esterified using any suitable esterification procedure, such as, for example, the industrial process comprising mixing with excess aqueous C₁-C₁₂ alkyl alcohol, using sulfuric acid as a catalyst under pressures of up to 791 kPa (100 psig) at 100°C-150°C, with residence times of generally less than 1 hour. In the case of MMA production, excess aqueous methanol is combined with the hydrolysis mixture. Esterification conditions are not critical and can be varied over a wide range. The only requirement is that the conditions be mild enough such that side reactions (e.g., dimethyl ether formation) and degradation products do not occur to an unacceptable extent.

The esterifying step produces an esterification mixture comprising MMA, α-MOB, and β-MEMOB along with significant quantities of water and unreacted methanol. The esterification mixture may also include other compounds, such as MAA and β-MOB. This mixture is subjected to one or more separation and/or purification steps, comprising the use of one or more distillation columns, to remove excess methanol, water, and light impurities, such as, without limitation, dimethyl ether. Generally, in accordance with the invention, liquid bottoms residue from at least one of the aforementioned distillation steps is further separated into an aqueous fraction and an organic fraction. For example, without limitation, fractional distillation conditions may be adjusted in a first distillation column to give a forerun of low boiling components such as water, unreacted methanol, small amounts of MMA, and the like and a bottoms stream rich in MMA and other higher boiling components such as α-MOB and β-MEMOB. Furthermore, the bottoms stream may be subjected to one or more further fractional distillation steps to produce a product grade MMA stream and a product column bottoms stream comprising MMA, as well as β-MOB, β-MEMOB, MAM, MAA, etc.

At least a portion of the organic fraction is then subjected to vaporization, along with a C₁-C₁₂ alkyl alcohol co-feed, such as, for example, without limitation, by a vaporizer as described hereinabove, to produce a vapor feed stream. The C₁-C₁₂ alkyl alcohol of the co-feed may be the same or different from the C₁-C₁₂ alkyl alcohol introduced in the esterifying step.

In particular, among the various fractions produced by the separation steps, at least one organic fraction comprising high purity MMA is obtained. This is a high purity MMA product-grade stream, whereas the remaining residue from this separation step is typically subjected to one or more further separation steps to obtain at least one organic fraction reduced in MMA content compared to the product stream. The organic fraction is then catalytically treated. The operating conditions suitable to effect such separations in the context of the method of the invention are well within the ability of persons of ordinary skill in the relevant art.

The process of obtaining the organic fraction typically includes a series of distillations wherein a crude MMA stream is obtained and refined by distilling overhead a purified, product-grade MMA stream. From this final product-grade distillation, a residue stream containing heavy ends results, which can be subjected to the recovery and catalytic conversion steps of the method in accordance with the present invention. This residue stream can be then vaporized to yield the vapor feed stream comprising residual MMA, α-MOB, β-MOB β-MEMOB, and MAA.

The vaporization step, involving vaporizing co-feed and at least a portion of the organic fraction, is accomplished by vaporizing, together or separately, the co-feed and at least a portion of the organic fraction. The vaporization may be performed in any apparatus suitable for vaporizing process streams comprising the constituents discussed hereinabove including, but not limited to, flash drums, shell-and-tube heat exchangers, plate-and-frame heat exchangers, natural or forced circulation evaporators, wiped film evaporators, or combinations thereof. In one embodiment of the invention, the vaporized stream is raised to the reaction temperature in the vaporizer. Suitable, but not limiting, conditions include operating pressures and temperatures in the respective ranges of 101 to 506 kPa absolute (1 to 5 atm) and 100 to 400°C. Preferably the pressure will be from 101 to 152 kPa absolute (1 to 1.5 atm) and the temperature will be from 250 to 360°C. The particular operating conditions are selected based upon the composition of the residue stream and are routinely determinable by persons of ordinary skill in the relevant art.

Once vaporized, the isobutyrate-containing components (i.e., α-MOB and β-MEMOB) of the vapor feed stream are converted in the presence of the catalyst to additional MMA.

The reaction step of the process comprises contacting a vapor feed stream from the vaporizing step with a catalyst under reaction conditions sufficient to convert by-products such as, for example, α-MOB, β-MEMOB, MAA and β-MOB, to additional MMA and produce a converted mixture that comprises MMA, MAA, C₁-C₁₂ alkyl alcohol, and water. In one embodiment of the invention, the aforesaid catalytic conversion is performed in the presence of methanol and/or a diluting agent such as an inert gas, at reaction temperatures of from about 200°C to about 400°C, preferably from 250 to 360°C. The reaction pressure is not particularly limited, and normally is equal to or slightly above atmospheric pressure for convenience of operation.

The product mixture from the reaction step can be subjected to distillation to recover the product C₁-C₁₂ alkyl methacrylate together with some light by-products such as C₁-C₁₂ alkyl isobutyrate and methacrylonitrile. The distillate containing the product C₁-C₁₂ alkyl methacrylate can recycled as desired to the process, e.g., to the separation and/or esterification steps.

One embodiment of the invention is a method for producing methacrylic acid esters comprising the steps of:
(1). hydrolyzing ACH with sulfuric acid to produce a hydrolysis mixture comprising 2-methacrylamide, α-sulfatoisobutyramide, α-hydroxyisobutyramide, and methacrylic acid;
(2). esterifying the hydrolysis mixture with a C₁-C₁₂ alkyl alcohol to produce an esterification mixture comprising a C₁-C₁₂ alkyl methacrylate, a C₁-C₁₂ alkyl α-hydroxyisobutyrate, and a C₁-C₁₂ alkyl β-C₁-C₁₂ alkoxyisobutyrate;
(3). separating the esterification mixture to produce an organic fraction comprising the C₁-C₁₂ alkyl methacrylate, the C₁-C₁₂ alkyl α-hydroxyisobutyrate and the C₁-C₁₂ alkyl β-C₁-C₁₂ alkoxyisobutyrate;
(4). separating the organic fraction to produce an enriched organic fraction comprising the C₁-C₁₂ alkyl methacrylate, the C₁-C₁₂ alkyl α-hydroxyisobutyrate and the C₁-C₁₂ alkyl β-C₁-C₁₂ alkoxyisobutyrate;
(5). flash distilling the enriched organic fraction to produce a vapor overhead stream comprising the C₁-C₁₂ alkyl methacrylate, the C₁-C₁₂ alkyl α-hydroxyisobutyrate and the C₁-C₁₂ alkyl β-C₁-C₁₂ alkoxyisobutyrate;
(6). condensing the vapor overhead stream to produce a vaporizer organic feed stream;
(7). providing a co-feed comprising a C₁-C₁₂ alkyl alcohol, which may or may not be the same alcohol as the C₁-C₁₂ alkyl alcohol used in the esterifying step (2);
(8). vaporizing the co-feed and at least a portion of the vaporizer organic feed stream to produce a vapor feed stream;
(9). contacting the vapor feed stream with the catalyst described hereinabove, to convert the C₁-C₁₂ alkyl α-hydroxyisobutyrate and C₁-C₁₂ alkyl β-C₁-C₁₂ alkoxyisobutyrate to additional C₁-C₁₂ alkyl methacrylate and produce a converted mixture that comprises methacrylic acid, the C₁-C₁₂ alkyl methacrylate, C₁-C₁₂ alkyl alcohol, and water.

Preferably, the vapor feed stream comprises both the vaporized co-feed and the vaporizer organic feed stream. However, it is also possible to separately feed vaporized co-feed and vaporizer organic feed stream to the vaporizer. Preferably, the vapor feed stream contains less than 25 wt.%, total of MAM and MMA dimer (dimethyl 5-methyl-2-methyleneadipate), based on the weight of the vapor feed stream, excluding co-feed.

Preferably, the vapor feed stream contains less than 85 wt.% total of MAM and MMA dimer, based on the weight of MAM and MMA dimer in the stream fed to the flash distillation apparatus.

This embodiment includes a flash distillation in the flash distillation apparatus. The flash distillation may be performed in any apparatus suitable for flash distilling process streams comprising the constituents discussed hereinabove. Suitable apparatus include, but are not limited to, flash drums, shell-and-tube heat exchangers, plate-and-frame heat exchangers, natural or forced circulation evaporators, wiped film evaporators, or combinations thereof. Suitable, but not limiting, conditions include operating pressures and temperatures in the respective ranges of 3.33-33.3 kPa (25-250 mmHg) and 100-200°C. Preferably, the pressure is kept as low as practical, such as 6.67 kPa (50 mmHg), to maintain a low corresponding temperature, such as less than or equal to 145°C. More preferably, the flash distillation pressure is in the range of 3.33-6.67 kPa (25-50 mm Hg) and the flash distillation temperature is maintained at less than 145°C. The vapor fraction may advantageously be from 0.1 to 1.0. The particular operating conditions are selected based upon the composition of the feed stream to the flash distillation and are routinely determinable by persons of ordinary skill in the relevant art to achieve the maximum recovery of desired components, while minimizing the heavies. In one embodiment of the invention, the flash distillation is a single stage flash distillation.

The bottoms stream from the flash distillation can be processed further, discarded as waste or burned as fuel.

The embodiment of the process that includes the flash distillation advantageously is operated in a manner that reduces fouling, reduces the buildup of heavy impurities in the recycle, reduces the organic fraction volume fed to the reactor and consequently the size of the reactor, and improves the energy efficiency and reliability of the product recovery process.

The mass yield of MMA can be calculated in two useful ways, as described in WO 2012/047883.

### SPECIFIC EMBODIMENTS OF THE INVENTION

The following examples are given to illustrate the invention and should not be construed as limiting its scope.

### Comparative Experiment 1 - Preparation of 10% Cs₂O/SiO₂ catalyst (Not an embodiment of the invention)

An aqueous solution is prepared by dissolving 2.72 grams of cesium acetate in 75 grams of deionized water. This solution is then added to a round bottom flask containing 18 grams of silica gel having a pore size of 150 angstrom (Davisil® Grade 636 silica gel commercially available from Aldrich). The mixture is stirred for 10 minutes and then subjected to rotary evaporation under vacuum to remove the water. The powder is further dried in a vacuum oven at room temperature overnight, followed by drying at 120°C for 4 hours and calcining at 450°C for 5 hours in a box furnace under an air atmosphere. The calcined powder contains a nominal 10 wt.% of Cs₂O and is designated 10% Cs₂O/SiO₂. It is then pressed and sieved into 14-20 mesh particles prior to being loaded into a fixed bed reactor for catalytic performance evaluation.

### Comparative Experiment 2 - Preparation of 10% Cs₂O/Bi/SiO₂ (Bi/Si=0.0014) catalyst (Not an embodiment of the invention)

A aqueous solution containing 0.174 g of Bi(NO₃)₃ ● 5H₂O and 50 g of deionized water is prepared. Then, 0.62g of 5 wt.% of nitric acid in water is added to the mixture to help dissolve the bismuth nitrate salt. The mixture is stirred at room temperature and then 2.27 g of cesium acetate is added. The solution is transferred into a round bottom flask containing 15 g of silica gel (Davisil® Grade 636 from Aldrich). The mixture is stirred for 10 minutes, followed by rotary evaporation at 50°C under vacuum to remove the water. The resulting powder is dried at 120°C for 5 hours and is calcined at 450°C for 5 hours in a box furnace under an air atmosphere. It is then pressed and sieved into 14-20 mesh size particles and designated 10% Cs₂O/Bi/SiO₂ (Bi/Si=0.0014), with a 0.0014 Bi/Si nominal atomic ratio.

### Comparative Experiment 3 - Preparation of 10% Cs₂O/Zr/SiO₂ (Zr/Si=0.01) catalyst (Not an embodiment of the invention)

An aqueous solution is prepared containing 0.58g of zirconyl nitrate [ZrO(NO₃)₂ ● xH₂O, from Arco Organics] and 2 g of 5 wt.% nitric acid aqueous solution in 62 g of deionized water. This solution is then added to a round bottom flask containing 15 g silica gel (Davisil® Grade 636 from Aldrich). The mixture is stirred for 10 minutes, followed by rotary evaporation at 50°C under vacuum to remove the water and further drying in a box furnace at 120°C for 2 hours. The dried mixture is mixed with an aqueous solution containing 50 g of water and 2.27 g of cesium acetate to form a slurry. The slurry is put on a rotary evaporator to remove water at 50°C under vacuum, followed by drying at 120°C for 5 hours and calcination at 450°C for 5 hours in a box furnace under an air atmosphere. It is then pressed and sieved into 14-20 mesh size particles and designated 10% Cs₂O/Zr/SiO₂ (Zr/Si=0.01), with a nominal atomic ratio of Zr/Si of 0.01.

### Example 4 - Preparation of 10% Cs₂O/B/SiO₂ (B/Si=0.00275) catalyst

An aqueous solution is prepared by dissolving 0.043g of boric acid in 50 g of deionized water. Then, 2.27 g of cesium acetate is added and dissolved into the solution. The resulting solution is then added into a round bottom flask containing 15 g silica gel (Davisil® Grade 636 from Aldrich). The mixture is stirred for 10 minutes, followed by rotary evaporation at 50°C under vacuum to remove the water and is further dried in a vacuum oven at room temperature overnight. The powder is further dried at 120°C for 5 hours and calcined at 450°C for 5 hours in a box furnace under an air atmosphere. It is then pressed and sieved into 14-20 mesh size particles and designated 10% Cs₂O/B/SiO₂ (B/Si=0.00275), with a nominal atomic ratio of B/Si of 0.00275.

### Example 5 - Preparation of 10% Cs₂O/B/SiO₂ (B/Si=0.041) catalyst

An aqueous solution is prepared by dissolving 0.64g of boric acid and 2.27 g of cesium acetate in 100 g of deionized water. This solution is added to a round bottom flask containing 15 g silica gel (Davisil® Grade 636 from Aldrich). The mixture is stirred for 10 minutes, followed by rotary evaporation at 50°C under vacuum to remove the water and the resulting powder is dried in a vacuum oven at room temperature overnight. The powder is further dried at 120°C for 5 hours and calcined at 450°C for 5 hours in a box furnace under an air atmosphere. It is then pressed and sieved into 14-20 mesh size particles and designated 10% Cs₂O/B/SiO₂ (B/Si=0.041), with a 0.041 B/Si nominal atomic ratio.

### Catalyst Evaluation

Catalyst, in the form of 14-20 mesh particles, is loaded into the middle of a ½" O.D. stainless steel plug flow tubular reactor with silicon carbide inert particles loaded above and below the catalyst charge. The amount of the catalyst charged is 1.0 to 3.0 g. The reactor tube is installed in an electrically heated clamshell furnace. The catalyst bed is pretreated in situ by flowing 40 sccm N₂ at 360°C to 370°C and 1 atmosphere pressure (1 bar) for 16-20 hours and then is cooled to the reaction temperature, typically 300°C-340°C, also at 1 atm(1 bar). Two different feed mixtures are tested.

"Feed A" is prepared by mixing 60 parts by weight of MMA residue distillate with 40 parts by weight of methanol. The distillate is obtained from the MMA product purification residue of the sulfuric acid hydrolysis of ACH. Distillation of the MMA product purification residue is achieved via continuous-flow fractional distillation using a 20-tray Oldershaw column. Reboiler and condenser pressures are respectively about 20.0 and 17.87 kPa (150 and 134 mmHg). 4-methoxyphenol (MeHQ) is added to the distillate as a polymerization inhibitor at a level of 15 ppm in the distillate. The final feed mixture composition, as measured by gas chromatography (GC), is shown in Table 1.

"Feed B" is prepared by mixing 50 parts by weight of the MMA residue distillate with 50 parts by weight of methanol. Distillation of the MMA product purification residue is achieved via continuous-flow flash evaporation at 6.67 kPa (50 mmHg) and 140°C. Phenothiazine is added to the distillate as a polymerization inhibitor at a level of 200 ppm in the distillate. The final feed mixture composition, as measured by GC, is shown in Table 1.

**Table 1: Reactor feed compositions**

| | Feed Composition (wt%)* | | | | | | | |
|---|---|---|---|---|---|---|---|---|
| Feed # | MeOH | MMA | α-MOB | β-MEMOB | β-MOB | MAA | MAM | MMA dimer |
| A | 40.79 | 1.24 | 44.94 | 12.1 | 0 | 0.008 | 0 | 0 |
| B | 48 | 0.149 | 23.77 | 7.11 | 1.68 | 5.75 | 3.6 | 5.89 |

| | | | | | | | | |
|---|---|---|---|---|---|---|---|---|
| * weight percentage from GC analysis. The balance are unknown compounds. | | | | | | | | |

Each feed (as a single liquid mixture) is delivered via syringe pump. The feed rate is 1.0 g/hr for each 1.0 g of catalyst loaded in order to maintain a weight hourly space velocity of 1.0 hr⁻¹. In some cases N₂ is co-fed in a separate line at 6 SCCM. In the case of co-feeding N₂, the liquid feed is combined with the co-feed before entering the reactor tube. The feed is injected directly into the top of the reactor tube, packed with inert SiC granules, and vaporized. The reactor effluent is swept through a cold trap submerged in an ice water bath to collect condensable products, which are weighed.

Feed and product stream compositions are measured by gas chromatography using two capillary columns connected in sequence (Column 1: Restek Rtx-1, dimensions 30 meters length x 0.53 millimeters ID x 1 micrometer (µm) film thickness; Column 2: Agilent DB-FFAP, dimensions 10 m length x 0.53 mm ID x 1 µm film thickness) and a flame ionization detector. Reaction product vapor exiting the cold trap is analyzed using a gas chromatograph equipped with silica gel and molecular sieve columns and a thermal conductivity detector.

Reactor temperature is varied initially to manipulate conversion. The stability test is started when an appropriate reaction temperature is identified at which the conversions of the major feed components, such as α-MOB and β-MEMOB, are each above 80% and preferably above 85%. The reaction temperature and feed rate are held constant during the stability test unless stated otherwise.

The concentration of MMA in the reactor exit stream is used to monitor the catalyst performance, as are the residual concentrations of alpha-MOB and beta-MEMOB.

The catalysts prepared according to Comparative Experiments 1, 2, and 3 are tested using Feed A and the conditions shown in Table 2. The concentrations of MMA, α-MOB and β-MEMOB in the reactor exit stream are shown in Table 3. The MMA concentration drops along with reaction time on stream, concurrently with increases in α-MOB and β-MEMOB concentrations. Hence, the catalysts deactivated.

**Table 2. Test conditions for catalysts of Comparative Experiments 1-3**

| Run # | C.E. 1 | C.E. 2 | C.E. 3 |
|---|---|---|---|
| Catalyst | 10% Cs₂O/SiO₂ | 10% Cs₂O/Bi SiO₂ (Bi/Si=0.0014) | 10% Cs₂O/Zr/SiO₂ (Zr/Si=0.01) |
| Dopant element | none | Bi | Zr |
| Feed #, feed rate | A, 1.50 g/hr | A, 1.5 g/hr | A, 1.5 g/hr |
| N₂ co-feed (SCCM) | 0 | 0 | 6 |
| Reaction Temperature* | 316°C | 332°C | 320°C |

| | | | |
|---|---|---|---|
| *R.T. = catalyst mid-bed temperature during stability test. | | | |

**Table 3. Concentrations of MMA, α-MOB and β-MEMOB in the reactor exit stream over the catalyst of C.E. 1 (10% Cs₂O/SiO₂)**

| **Time on Stream (hour)** | **Component concentration in reactor effluent (wt%)** | | |
|---|---|---|---|
| | **MMA** | **A-MOB** | **B-MEMOB** |
| 4.17 | 43.33 | 1.52 | 0.73 |
| 26.85 | 40.85 | 2.32 | 1.59 |
| 50.63 | 38.78 | 3.31 | 2.63 |
| 74.37 | 37.89 | 3.84 | 3.06 |
| 111.26 | 36.98 | 4.52 | 3.44 |

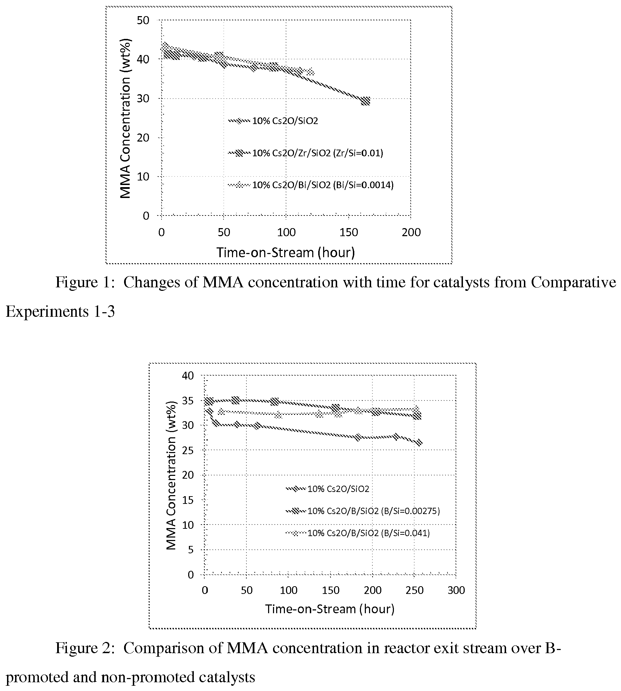

The changes of MMA concentration with time among catalysts from Comparative Experiments 1-3 are shown in Figure 1. While the reaction proceeds, it is clear that the introduction of the Bi and Zr dopants does not improve the catalyst stability against deactivation.

It is observed that the concentration of the desired product MMA drops slowly with reaction time during the dehydration process over Cs₂O/SiO₂ catalyst, which is attributed to the deactivation of the catalyst as indicated by the drop in conversions of the major feed components. Therefore, a frequent regeneration is required to recover the catalyst performance. The stability of the catalyst against rapid deactivation is highly desirable to reduce the frequency of the catalyst regeneration and to maintain high value recovery from MMA distillation residue.

The B-promoted catalysts of Examples 4-5 perform dramatically better. The test conditions for the B-promoted catalysts and the un-promoted catalyst are listed in Table 4. Feed "B" is used in these tests. The MMA concentration in the reactor exit stream is shown in Figure 2. The B-promoted catalysts show significantly improved stability against deactivation. The high initial MMA concentration is maintained for a much longer period of time over B-promoted catalysts compared to the non-promoted catalyst.

**Table 3. Test conditions for B-promoted catalysts and non-promoted catalyst.**

| Run # | C.E. 1 | Ex. 4 | Ex. 5 |
|---|---|---|---|
| Catalyst | 10% Cs₂O/SiO₂ | 10% Cs₂O/B/SiO₂ (B/Si=0.00275) | 10% Cs₂O/B/SiO₂ (B/Si=0.041) |
| Dopant element | none | B | B |
| Feed #, feed rate | B, 1.0 g/hr | B, 1.0 g/hr | B, 1.0 g/hr |
| N₂ co-feed (SCCM) | 0 | 0 | 0 |
| Reaction conditions* | 320-327°C | 333°C | 342°C |

| | | | |
|---|---|---|---|
| *The temperature is increased from 320°C (0 - 8 hours time-on-stream), then to 323°C (8 - 14.7 hours), and eventually to 327°C (14.7 - 258 hours) to compensate the rapid drop of the conversions for α-MOB and β-MEMOB initially. | | | |

The results surprisingly demonstrate that small amounts of boron significantly improves the catalyst stability.

## Claims

1. A method for producing methacrylic acid esters comprising the steps of:
(d) providing a C₁-C₁₂ alkyl alcohol and an organic fraction comprising C₁-C₁₂ alkyl methacrylate, C₁-C₁₂ alkyl α-hydroxyisobutyrate and C₁-C₁₂ alkyl β-C₁-C₁₂ alkoxyisobutyrate;
(e) vaporizing at least a portion of the organic fraction and at least a portion of the C₁-C₁₂ alkyl alcohol;
(f) contacting the vaporized organic fraction and alcohol with a catalyst to convert the C₁-C₁₂ alkyl α-hydroxyisobutyrate and C₁-C₁₂ alkyl β-C₁-C₁₂ alkoxyisobutyrate to additional C₁-C₁₂ alkyl methacrylate and produce a converted mixture that comprises a C₁-C₁₂ alkyl methacrylate, methacrylic acid, C₁-C₁₂ alkyl alcohol, and water, wherein the catalyst (1) comprises at least one element selected from the group consisting of lithium, sodium, potassium, rubidium, cesium and francium, (2) is supported on a support comprising porous silica, and (3) comprises from 0.005 to 5 moles of boron as a promoter per 100 moles of silica.

2. The process of Claim 1 wherein the amount of boron is from 0.010 to 4 moles per 100 moles of silica.

3. The process of any of the preceding claims wherein the amount of boron is from 0.1 to 1 moles per 100 moles of silica.

4. The process of any of Claim 1 wherein the amount of boron is from 0.005 to less than 0.25 moles per 100 moles of silica.

5. The process of any of the preceding claims, further comprising the steps:
(a) hydrolyzing ACH with sulfuric acid to produce a hydrolysis mixture comprising 2-methacrylamide, α-sulfatoisobutyramide, α-hydroxyisobutyramide, and methacrylic acid;
(b) esterifying the hydrolysis mixture with a C₁-C₁₂ alkyl alcohol to produce an esterification mixture comprising a C₁-C₁₂ alkyl methacrylate, a C₁-C₁₂ alkyl α-hydroxyisobutyrate and C₁-C₁₂ alkyl β- C₁-C₁₂ alkoxyisobutyrate;
(c) separating the esterification mixture into an aqueous fraction and an organic fraction comprising C₁-C₁₂ alkyl methacrylate, C₁-C₁₂ alkyl α-hydroxyisobutyrate and C₁-C₁₂ alkyl β-C₁-C₁₂ alkoxyisobutyrate.

6. The process of any of the preceding claims wherein the support is a porous silica support and is selected from the group consisting of silica gel, fumed silica, colloidal silica, mesoporous silica, foam silica, and combinations thereof.

7. The process of any of the preceding claims wherein the support is a porous silica support and is selected from the group consisting of silica gel, fumed silica, colloidal silica, and combinations thereof.

8. The process of any of the preceding claims, further comprising step (d2) that comprises flash distilling the organic fraction to separate it into a stripped heavy residue stream and a flash vapor overhead stream, then feeding at least a portion of the flash overhead stream to step (e) as the organic fraction.

9. The process of any of the preceding claims wherein the porous silica support has an average pore size of at least 1 nanometer.

10. The process of any of the preceding claims wherein the C₁-C₁₂ alkyl alcohol is methanol, the C₁-C₁₂ alkyl methacrylate is methyl methacrylate, the C₁-C₁₂ alkyl α-hydroxyisobutyrate is methyl α-hydroxyisobutyrate (a-MOB), and the C₁-C₁₂ alkyl β-C₁-C₁₂ alkoxyisobutyrate is methyl β-methoxyisobutyurate (β-MEMOB).

## Patentansprüche

1. Eine Methode zur Herstellung von Methacrylsäureestern, das die folgenden Schritte beinhaltet:
(d) Bereitstellen eines C₁-C₁₂-Alkylalkohols und einer organischen Fraktion, die C₁-C₁₂-Alkylmethacrylat, C₁-C₁₂-Alkyl-α-hydroxyisobutyrat und C₁-C₁₂-Alkyl-β-C₁-C₁₂-Alkoxyisobutyrat beinhaltet;
(e) Verdampfen mindestens eines Teils der organischen Fraktion und mindestens eines Teils des C₁-C₁₂-Alkylalkohols;
(f) In-Kontakt-Bringen der verdampften organischen Fraktion und des Alkohols mit einem Katalysator, um das C₁-C₁₂-Alkyl-α-hydroxyisobutyrat und C₁-C₁₂-Alkyl-β-C₁-C₁₂-Alkoxyisobutyrat in zusätzliches C₁-C₁₂-Alkylmethacrylat umzuwandeln und ein umgewandeltes Gemisch herzustellen, das ein C₁-C₁₂-Alkylmethacrylat, Methacrylsäure, C₁-C₁₂-Alkylalkohol und Wasser beinhaltet, wobei der Katalysator (1) mindestens ein Element, ausgewählt aus der Gruppe, bestehend aus Lithium, Natrium, Kalium, Rubidium, Cäsium und Francium beinhaltet, (2) auf einem Träger getragen wird, der poröses Siliciumdioxid beinhaltet, und (3) 0,005 bis 5 Mol Bor als einen Promotor pro 100 Mol Siliciumdioxid beinhaltet.

2. Verfahren gemäß Anspruch 1, wobei die Menge an Bor 0,010 bis 4 Mol pro 100 Mol Siliciumdioxid beträgt.

3. Verfahren gemäß einem der vorhergehenden Ansprüche, wobei die Menge an Bor 0,1 bis 1 Mol pro 100 Mol Siliciumdioxid beträgt.

4. Verfahren gemäß Anspruch 1, wobei die Menge an Bor zwischen 0,005 bis weniger als 0,25 Mol pro 100 Mol Siliciumdioxid beträgt.

5. Verfahren gemäß einem der vorhergehenden Ansprüche, das ferner die folgenden Schritte beinhaltet:
(a) Hydrolysieren von ACH mit Schwefelsäure, um ein Hydrolysegemisch herzustellen, das 2-Methacrylamid, α-Sulfatoisobutyramid, α-Hydroxyisobutyramid und Methacrylsäure beinhaltet;
(b) Verestern des Hydrolysegemisches mit einem C₁-C₁₂-Alkylalkohol zur Herstellung eines Veresterungsgemisches, das ein C₁-C₁₂-Alkylmethacrylat, ein C₁-C₁₂-Alkyl-α-hydroxyisobutyrat und C₁-C₁₂-Alkyl-β-C₁-C₁₂-alkoxyisobutyrat beinhaltet;
(c) Trennen des Veresterungsgemisches in eine wässrige Fraktion und eine organische Fraktion, die C₁-C₁₂-Alkylmethacrylat, C₁-C₁₂-Alkyl-α-hydroxyisobutyrat und C₁-C₁₂-Alkyl-α-C₁-C₁₂-Alkoxyisobutyrat beinhaltet.

6. Verfahren gemäß einem der vorhergehenden Ansprüche, wobei der Träger ein poröser Siliciumdioxidträger ist und aus der Gruppe ausgewählt ist, die aus Silicagel, pyrogenem Siliciumdioxid, kolloidalem Siliciumdioxid, mesoporösem Siliciumdioxid, Siliciumdioxidschaum und Kombinationen davon besteht.

7. Verfahren gemäß einem der vorhergehenden Ansprüche, wobei der Träger ein poröser Siliciumdioxidträger ist und aus der Gruppe ausgewählt ist, die aus Silicagel, pyrogenem Siliciumdioxid, kolloidalem Siliciumdioxid und Kombinationen davon besteht.

8. Verfahren gemäß einem der vorhergehenden Ansprüche, ferner beinhaltend den Schritt (d2), der das Entspannungsverdampfen der organischen Fraktion beinhaltet, um sie in einen abgelösten schweren Rückstandsstrom und einen Entspannungsdampf-Überkopfstrom zu trennen und dann mindestens einen Teil des Entspannungs-Überkopfstroms Schritt (e) als die organische Fraktion zuzuführen.

9. Verfahren gemäß einem der vorhergehenden Ansprüche, wobei der poröse Siliciumdioxidträger eine durchschnittliche Porengröße von mindestens 1 Nanometer aufweist.

10. Verfahren gemäß einem der vorhergehenden Ansprüche, wobei der C₁-C₁₂-Alkylalkohol Methanol ist, das C₁-C₁₂-Alkylmethacrylat Methylmethacrylat ist, das C₁-C₁₂-Alkyl-α-hydroxyisobutyrat Methyl-α-hydroxyisobutyrat (a-MOB) ist und das C₁-C₁₂-Alkyl-α-C₁-C₁₂-Alkoxyisobutyrat Methyl-β-methoxyisobutyrat (β-MEMOB) ist.

## Revendications

1. Une méthode de production d'esters d'acide méthacrylique comprenant les étapes consistant à :
(d) fournir un alcool C₁-C₁₂ alkylique et une fraction organique comprenant un C₁-C₁₂ alkyl méthacrylate, un C₁-C₁₂ alkyl α-hydroxyisobutyrate et un C₁-C₁₂ alkyl β-C₁-C₁₂ alcoxyisobutyrate;
(e) vaporiser au moins une portion de la fraction organique et au moins une portion de l'alcool C₁-C₁₂ alkylique ;
(f) mettre en contact la fraction organique et l'alcool vaporisés avec un catalyseur afin de convertir le C₁-C₁₂ alkyl α-hydroxyisobutyrate et le C₁-C₁₂ alkyl β-C₁-C₁₂ alcoxyisobutyrate en un C₁-C₁₂ alkyl méthacrylate supplémentaire et de produire un mélange converti qui comprend un C₁-C₁₂ alkyl méthacrylate, de l'acide méthacrylique, un alcool C₁-C₁₂ alkylique, et de l'eau, dans laquelle le catalyseur (1) comprend au moins un élément sélectionné dans le groupe constitué du lithium, du sodium, du potassium, du rubidium, du césium et du francium, (2) est supporté sur un support comprenant de la silice poreuse, et (3) comprend de 0,005 à 5 moles de bore en tant que promoteur pour 100 moles de silice.

2. Le procédé de la revendication 1 dans lequel la quantité de bore va de 0,010 à 4 moles pour 100 moles de silice.

3. Le procédé de n'importe lesquelles des revendications précédentes dans lequel la quantité de bore va de 0,1 à 1 mole pour 100 moles de silice.

4. Le procédé de la revendication 1 dans lequel la quantité de bore va de 0,005 à moins de 0,25 mole pour 100 moles de silice.

5. Le procédé de n'importe lesquelles des revendications précédentes, comprenant en outre les étapes consistant à :
(a) hydrolyser de l'ACH avec de l'acide sulfurique afin de produire un mélange d'hydrolyse comprenant du 2-méthacrylamide, de l'a-sulfatoisobutyramide, de l'a-hydroxyisobutyramide, et de l'acide méthacrylique ;
(b) estérifier le mélange d'hydrolyse avec un alcool C₁-C₁₂ alkylique afin de produire un mélange d'estérification comprenant un C₁-C₁₂ alkyl méthacrylate, un C₁-C₁₂ alkyl α-hydroxyisobutyrate et un C₁-C₁₂ alkyl β-C₁-C₁₂ alcoxyisobutyrate ;
(c) séparer le mélange d'estérification en une fraction aqueuse et en une fraction organique comprenant le C₁-C₁₂ alkyl méthacrylate, le C₁-C₁₂ alkyl α-hydroxyisobutyrate et le C₁-C₁₂ alkyl β-C₁-C₁₂ alcoxyisobutyrate.

6. Le procédé de n'importe lesquelles des revendications précédentes dans lequel le support est un support en silice poreuse et est sélectionné dans le groupe constitué de gel de silice, de silice pyrogénée, de silice colloïdale, de silice mésoporeuse, de silice mousse, et de combinaisons de ceux-ci.

7. Le procédé de n'importe lesquelles des revendications précédentes dans lequel le support est un support en silice poreuse et est sélectionné dans le groupe constitué de gel de silice, de silice pyrogénée, de silice colloïdale, et de combinaisons de ceux-ci.

8. Le procédé de n'importe lesquelles des revendications précédentes, comprenant en outre l'étape (d2) qui comprend la distillation éclair de la fraction organique afin de la séparer en un courant de résidus lourds rectifié et en un courant de tête de vapeur éclair, puis l'introduction d'au moins une portion du courant de tête éclair au niveau de l'étape (e) en tant que fraction organique.

9. Le procédé de n'importe lesquelles des revendications précédentes dans lequel le support en silice poreuse a une taille de pore moyenne d'au moins 1 nanomètre.

10. Le procédé de n'importe lesquelles des revendications précédentes dans lequel l'alcool C₁-C₁₂ alkylique est le méthanol, le C₁-C₁₂ alkyl méthacrylate est le méthyl méthacrylate, le C₁-C₁₂ alkyl α-hydroxyisobutyrate est le méthyl α-hydroxyisobutyrate (α-MOB), et le C₁-C₁₂ alkyl β-C₁-C₁₂ alcoxyisobutyrate est le méthyl β-méthoxyisobutyrate (β-MEMOB).
